# EUROPÄISCHE PATENTSCHRIFT

(11) **EP 0 382 053 B1**
(45) Veröffentlichungstag und Bekanntmachung des Hinweises auf die Patenterteilung: **22.09.1993**
(21) Anmeldenummer: 90101789.7
(22) Anmeldetag: 30.01.1990
(51) Int. Cl.: C07C 231/02, C07C 235/84

(54) **Verfahren zur Herstellung von Anthrachinonderivaten**
Process for the preparation of anthraquinone derivatives
Procédé pour la fabrication de dérivés d'anthraquinone

(30) Priorität: 08.02.1989 DE 3903623
(43) Veröffentlichungstag der Anmeldung: 16.08.1990
(73) Patentinhaber: BASF Aktiengesellschaft, 67063 Ludwigshafen (DE)
(72) Erfinder: Hoch, Helmut, Dr., D-6719 Weisenheim (DE); Kilpper, Gerhard, Dr., D-6719 Carlsberg (DE)

(56) Entgegenhaltungen:
- EP-A- 0 189 772
- EP-A- 0 326 866
- DE-C- 475 687
- DE-C- 604 279
- DE-C- 1 277 477
- DE-C- 1 278 046
- FR-A- 2 208 905
- FIAT REPORT 1313 II Seite 157
- COMPREHENSIVE ORGANIC CHEMISTRY, D. Barton et al, Pergamon Press, vol. 2, page 960

## Beschreibung

Die vorliegende Erfindung betrifft ein verbessertes Verfahren zur Herstellung von Anthrachinonderivaten der allgemeinen Formel I

(A¹-CO-NH-)ₙA² I

in der A¹ und A² Anthrachinon-Reste und/oder anthrachinoide Reste bedeuten und n für 1 oder 2 steht, durch Umsetzung der Anthrachinoncarbonsäuren oder ihrer Derivate der allgemeinen Formel II

A¹-COX II

in der X für eine Hydroxylgruppe oder ein Chlor- oder Bromatom steht, mit einem Aminoanthrachinon der allgemeinen Formel III

A²(NH₂)ₙ III

in Gegenwart einer tertiären Stickstoffbase.

Die Carbonsäureamide I stellen wichtige Vorprodukte für Küpenfarbstoffe, beispielsweise Colour Index (C.I.) Vat Red 10 (C.I. No. 67000) und C.I. Vat Blue 31 (C.I. No. 67105) oder selbst Küpenfarbstoffe dar, beispielsweise C.I. Vat Red 21 (C.I. No. 61670).

Aus der Literaturstelle Comprehensive Organic Chemistry, D. Barton und W.D. Ollis, p. 960, Pergamon Press, ist bereits bekannt, daß bei der Acylierung von Aminen mit Carbonsäurehalogeniden die Gegenwart von tertiären Aminen vorteilhaft ist, um die Säure zu entfernen.

Die Carbonsäureamide I sind bekannt und nach dem Verfahren der DE-C 475 687 in der Weise erhältlich, daß man Anthrachinonaldehyde, Anthrachinoncarbonsäuren oder Derivate hiervon mit Aminoanthrachinonen umsetzt. In dieser Patentschrift wird zur Bindung der dem Reaktionsprodukt aus 1-Aminoanthrachinon-2-carbonsäurechlorid und 2-Amino-3-hydroxyanthrachinon noch anhaftenden Salzsäure die Zugabe von unter anderem Dimethylanilin als säurebindenden oder abstumpfendem Mittel empfohlen.

Die Literaturstelle FIAT Report 1313 II, S. 157 beschreibt die Umsetzung von 1-Nitroanthrachinon-2-carbonsäurechlorid mit 2-Amino-3-hydroxyanthrachinon in Gegenwart von katalytischen Mengen Pyridin.

Die ältere deutsche Patentanmeldung P 38 02 613 betrifft die Herstellung von Acylaminen der Anthrachinonreihe aus substituierten Anthrachinoncarbonsäurehalogeniden und Aminoanthrachinonen in Benzoesäuremethylester als Lösungsmittel in Gegenwart der tertiären Stickstoffbase Chinolin, Pyridin oder Triethylamin.

Da die Ausbeuten und die Reinheiten der nach dem angeführten Stand der Technik hergestellten Verfahrensprodukte I jedoch zu wünschen übrig lassen, was sich nachteilig auf die Qualität der resultierenden Farbstoffe auswirkt, war es die Aufgabe der vorliegenden Erfindung , diese Nachteile zu beseitigen.

Demgemäß wurde das eingangs definierte Verfahren zur Herstellung von Anthrachinonderivaten gefunden, welches dadurch gekennzeichnet ist, daß man die folgenden tertiären Stickstoffbasen verwendet:
(a) Trialkylamine mit drei gleichen oder verschiedenen nichtcyclischen Alkylresten mit der Ausnahme von Triethylamin im Falle der Umsetzung in Benzoesäuremethylester als Lösungsmittel, beispielsweise Triethylamin, ausgenommen bei Verwendung von Benzoesäuremethylester als Lösungsmittel, Tri-n-propylamin, Triisopropylamin, Tri-n-butylamin, Triisobutylamin, Tri-n-pentylamin, Tri-n-hexylamin, Tri-n-heptylamin, Tri-n-octylamin, Diisopropylmethylamin und Diisopropylethylamin,
(b) Trialkylamine mit 1,2 oder 3 Cyclopentyl- und/oder Cyclohexylresten, beispielsweise N,N-Dimethylcyclopentylamin, N,N-Dimethylcyclohexylamin, N,N-Diethylcyclohexylamin, N,N-Di-n-propylcyclohexylamin, N,N-Diisopropylcyclohexylamin, N,N-Di-n-butylcyclohexylamin, N,N-Diisobutylcyclohexylamin, N-Methyldicyclohexylamin, N-Ethyldicyclohexylamin, N-(n-Propyl)dicyclohexylamin, N-Isopropyldicyclohexylamin, N-(n-Butyl)dicyclohexylamin, N-Isobutyldicyclohexylamin und Tricyclohexylamin oder
(c) Aralkylamine mit einem Benzylrest, beispielsweise N,N-Dimethylbenzylamin, N,N-Diethylbenzylamin, N,N-Di-n-propylbenzylamin, N,N-Diisopropylbenzylamin, N,N-Di-n-butylbenzylamin, N,N-Diisobutylbenzylamin, N-Ethyl-N-methylbenzylamin, N-Methyl-N-propylbenzylamin und N-Methyl-N-butyl-benzylamin.

Besonders gute Ergebnisse erzielt man mit N,N-Dimethylcyclohexylamin, Tri-n-butylamin und N,N-Dimethylbenzylamin.

Diese tertiären Stickstoffbasen werden im erfindungsgemäßen Verfahren in einer Menge von 0,01 bis 1 mol pro Mol II, vorzugsweise 0,01 bis 0,5 mol pro Mol II, insbesondere 0,05 bis 0,5 mol pro Mol II, eingesetzt.

Die Anthrachinoncarbonsäuren oder ihre Säurehalogenide II können an ihrem Anthrachinon-Rest oder anthrachinoidem Rest A¹ zusätzliche Substituenten tragen, beispielsweise C₁- bis C₄-Alkylgruppen, gegebenenfalls durch C₁- bis C₄-Alkylgruppen substituierte Aminogruppen, Nitrogruppen, Chlor- oder Bromatome. Von besonderem Interesse sind diejenigen Verbindungen II, welche die gegebenenfalls substituierte Aminogruppe am 1-C-Atom und die Carboxylgruppe am 2-C-Atom des Anthrachinongerüstes aufweisen. Ein weiterer Substituent befindet sich dann bevorzugt am 4-C-Atom.

Als Beispiele für II können 1-Aminoanthrachinon-2-carbonsäure, 1-(N,N-Dimethylamino)anthrachinon-2-carbonsäure, 1-Amino-4-methylanthrachinon-2-carbonsäure, 1-Amino-4-nitroanthrachinon-2-carbonsäure, 1-Amino-4-chloranthrachinon-2-carbonsäure, 1-Nitroanthrachinon-2-carbonsäure und als anthrachinoides System 1,9-Anthrapyrimidin-2-carbonsäure (1,9-Pyrimidino-10-anthron-2-carbonsäure) genannt werden.

Die Aminoanthrachinone III können ebenfalls an ihrem Anthrachinon-Rest oder anthrachinoidem Rest A² zusätzliche Substituenten tragen, zum Beispiel C₁- bis C₄-Alkylgruppen, Hydroxylgruppen, Benzoylreste, Chlor- oder Bromatome.

Als Aminoanthrachinone III kommen beispielsweise 1-Aminoanthrachinon, 2-Aminoanthrachinon, 2-Amino-3-methylanthrachinon, 2-Amino-3-bromanthrachinon, 2-Amino-3-hydroxyanthrachinon, 1,4-Diaminoanthrachinon, 1,5-Diaminoanthrachinon, 1-Amino-4-benzoylanthrachinon und 1-Amino-5-benzoylanthrachinon in Betracht.

Als Lösungsmittel für das erfindungsgemäße Verfahren setzt man in der Regel Nitrobenzol, o-Dichlorbenzol oder Benzoesäuremethylester ein.

Das erfindungsgemäße Verfahren wird normalerweise so durchgeführt, daß die Anthrachinoncarbonsäure oder ihr Säurehalogenid II in einem dieser Lösungsmittel zusammen mit einer der genannten tertiären Stickstoffbasen vorgelegt wird. Im Falle einer Carbonsäure (X=OH) wird zusätzlich bei Raumtemperatur ein säurehalogenidbildendes Reagenz, beispielsweise Thionylchlorid, Phosphortrichlorid, Phosphortribromid, Phosphorpentachlorid oder Phosphorpentabromid, zugegeben und auf üblicherweise 80 bis 110°C erwärmt, bis die Carbonsäure vollständig umgesetzt ist. Anschließend gibt man das Aminoanthrachinon III zu der Lösung des Anthrachinoncarbonsäurehalogenids. Die Kondensation zum Carbonsäureamid I wird zweckmäßigerweise bei 130 bis 160°C vorgenommen. Nach Abkühlen des Reaktionsansatzes wird die Aufarbeitung in der üblichen Weise durchgeführt, beispielsweise durch Filtrieren oder durch Eindampfen des Reaktionsgemisches zur Trockene.

In einer bevorzugten Ausführungsform des erfindungsgemäßen Verfahrens wird die Anthrachinoncarbonsäure oder ihr Säurehalogenid II zusammen mit dem Aminoanthrachinon III und der tertiären Stickstoffbase in einem der genannten Lösungsmittel vorgelegt. Im Falle einer Carbonsäure (X=OH) wird zusätzlich bei Raumtemperatur mit einem säurehalogenidbildenden Reagenz, beispielsweise Thionylchlorid, Phosphortrichlorid, Phosphortribromid, Phosphorpentachlorid oder Phosphorpentabromid, versetzt. Danach erwärmt man langsam auf die zur Kondensation zum Carbonsäureamid erforderliche Temperatur von zweckmäßigerweise 130 bis 160°C. Nach Abkühlen des Reaktionsansatzes wird die Aufarbeitung in der üblichen Weise vorgenommen. Die erfindungsgemäße Umsetzung kann natürlich auch unter Druck in geschlossenen Gefäßen vorgenommen werden.

Durch Destillation können aus den bei der Aufarbeitung anfallenden Mutterlaugen vorteilhafterweise die eingesetzte tertiäre Stickstoffbase und das Lösungsmittel leicht zurückgewonnen und wiederverwendet werden.

Mit den erfindungsgemäß zu verwendenden tertiären Stickstoffbasen erhält man Carbonsäureamide in verbesserten Ausbeuten und in höheren Reinheiten im Vergleich zu den aus dem Stand der Technik bekannten aromatischen Aminen wie Dimethylanilin oder heteroaromatischen Verbindungen mit einem Ringstickstoffatom wie Pyridin und Chinolin. Die höheren Reinheiten bewirken bei den resultierenden Farbstoffen farbstärkere Färbungen und deutlich reinere Farbtöne. Insbesondere erzielt man mit dem erfindungsgemäßen Verfahren deutlich bessere Raum-Zeit-Ausbeuten, weil man den Lösungsmittelanteil der Reaktionsmischung, bezogen auf die Einsatzstoffe, stark senken kann.

### Beispiele

Die angegebenen Werte für die Raum-Zeit-Ausbeuten geben den Quotienten aus der erhaltenen Produktmenge in kg und dem Volumen des Reaktionsgemisches in l an und beziehen sich jeweils auf einen Ansatz.

### Beispiel 1

In 560 kg o-Dichlorbenzol, aus dem zuvor unter Durchleiten von Stickstoff das Restwasser durch Erhitzen auf 140°C abdestilliert worden war, wurden bei Raumtemperatur 67,7 kg 1-Aminoanthrachinon-2-carbonsäure, 78,4 kg 2-Amino-3-bromanthrachinon und 11,2 kg N,N-Dimethylcyclohexylamin (entsprechend 0,35 mol pro Mol der Carbonsäure) eingetragen. Nach Zugabe von 39 kg Thionylchlorid wurde das Gemisch innerhalb von 5 Stunden langsam auf 145°C erwärmt und 3 Stunden bei dieser Temperatur gehalten. Nach Abkühlung auf 110°C wurde das Produkt abfiltriert, mit o-Dichlorbenzol, dann mit Methanol und schließlich mit Wasser gewaschen und bei 120 °C im Vakuum getrocknet.

Man erhielt 129 kg des entsprechenden Carbonsäureamids mit einem Reinproduktgehalt von 98,8 Gew.%; die Gehaltsbestimmung erfolgte durch Umsetzung zum Farbstoff C.I. Vat Red 10 (C.I. No. 67000) nach dem in der DE-A 22 59 329, Beispiel 5, beschriebenen Verfahren und photometrische Bestimmung dieses Farbstoffes. Die Ausbeute für die Umsetzung zum Carbonsäureamid betrug 91 %, die Raum-Zeit-Ausbeute lag hier bei 0,228 kg/l.

Wurde die gleiche Umsetzung zum Carbonsäureamid mit 0,35 mol N,N-Dimethylbenzylamin pro Mol der eingesetzten Carbonsäure anstelle des N,N-Dimethylcyclohexylamins durchgeführt, lag die erzielte Ausbeute bei 88% und die Raum-Zeit-Ausbeute bei 0,22 kg/l.

Die Aufarbeitung des Reaktionsgemisches durch Eindampfen zur Trockene nach beendeter Reaktion und weitere Umsetzung des Rückstandes zum Farbstoff C.I. Vat Red 10 ohne weitere Reinigung des Carbonsäureamids führte sowohl bei Verwendung von N,N-Dimethylcyclohexylamin als auch von N,N-Dimethylbenzylamin zu den gleichen Resultaten.

### Beispiel 2 (Vergleichbeispiel)

Wurde die gleiche Umsetzung wie in Beispiel 1 mit 0,35 mol Pyridin oder Chinolin pro Mol der eingesetzten Carbonsäure anstelle des N,N-Dimethylcyclohexylamins oder des N,N-Dimethylbenzylamins durchgeführt, konnten in der vorgelegten Menge von 560 kg o-Dichlorbenzol bestenfalls 48,4 kg 1-Aminoanthrachinon-2-carbonsäure und 56 kg 2-Amino-3-bromanthrachinon umgesetzt werden, da sonst das heiße Reaktionsgemisch nicht mehr rührfähig war.

Die Ausbeute betrug hierbei im günstigsten Fall 82 %, die entsprechende Raum-Zeit-Ausbeute lag bei 0,146 kg/l. Eine Färbung mit dem gemäß DE-A 22 59 329, Beispiel 5, aus den erhaltenen Carbonsäureamiden jeweils hergestellten Farbstoff C.I. Vat Red 10 war um 5 bis 10% farbschwächer und im Farbton deutlich unreiner als eine Färbung mit dem gleichen Farbstoff aus Beispiel 1.

### Beispiel 3

In 350 kg Nitrobenzol, aus dem zuvor unter Durchleiten von Stickstoff das Restwasser durch Erhitzen auf 140°C abdestilliert worden war, wurden bei Raumtemperatur 40 kg 1-Amino-4-nitroanthrachinon-2-carbonsäure, 36 kg 2-Amino-3-bromanthrachinon und 4,0 kg Tri-n-butylamin (entsprechend 0,17 mol pro Mol der Carbonsäure) eingetragen. Nach der Zugabe von 25 kg Thionylchlorid wurde innerhalb von 4,5 Stunden die Temperatur langsam auf 145°C erhöht und das Reaktionsgemisch 2 Stunden bei dieser Temperatur gehalten. Nach Abkühlung auf 80°C wurde das Produkt abfiltriert, mit Nitrobenzol, dann mit Methanol und schließlich mit Wasser gewaschen und bei 120°C im Vakuum getrocknet.

Man erhielt 66 kg des entsprechenden Carbonsäureamids mit einem Reinproduktgehalt von 98,5 Gew.%; die Gehaltsbestimmung erfolgte durch Säulenchromatographie. Die Ausbeute betrug 85%, die Raum-Zeit-Ausbeute lag bei 0,216 kg/l.

### Beispiel 4 (Vergleichsbeispiel)

Wurde die gleiche Umsetzung wie in Beispiel 3 mit 0,17 mol Pyridin pro Mol der eingesetzten Carbonsäure anstelle des Trinbutylamins durchgeführt, lag die erhaltene Ausbeute bei 76% und die Raum-Zeit-Ausbeute bei 0,12 kg/l.

### Beispiel 5

Wurde die gleiche Umsetzung wie in Beispiel 3 mit der äquivalenten Menge 1-Nitroanthrachinon-2-carbonsäure anstelle der 1-Amino-4-nitroanthrachinon-2-carbonsäure durchgeführt, betrug sowohl bei Verwendung von 0,17 mol Tri-n-butylamin als auch von 0,17 mol N,N-Dimethylcyclohexylamin pro Mol der Carbonsäure die erzielte Ausbeute 90 % und die Raum-Zeit-Ausbeute 0,22 kg/l. Das erhaltene Carbonsäureamid hatte eine Säulenchromatographisch bestimmte Reinheit von 98 %.

### Beispiel 6

In 250 kg Benzoesäuremethylester, aus dem zuvor unter Durchleiten von Stickstoff das Restwasser durch Erhitzen auf 140°C abdestilliert worden war, wurden bei Raumtemperatur 20 kg 1-Aminoanthrachinon-2-carbonsäure, 0,5 kg N,N-Dimethylcyclohexylamin (entsprechend 0,05 mol pro Mol der Carbonsäure) und 11 kg Thionylchlorid eingebracht. Danach wurde innerhalb von einer Stunde auf 105°C erwärmt und eine Stunde bei dieser Temperatur gehalten. Nach Wiederabkühlung auf 70°C wurden 8,0 kg 1,4-Diaminoanthrachinon eingetragen. Anschließend wurde innerhalb von 3 Stunden auf 145°C erwärmt und 2 Stunden bei dieser Temperatur gehalten. Nach Abkühlung auf 80°C wurde das Produkt abfiltriert, mit Methanol und danach mit Wasser gewaschen und bei 120 °C im Vakuum getrocknet.

Man erhielt 27 kg des entsprechenden Biscarbonsäureamids, welches gleichzeitig den Farbstoff C.I. Vat Red 21 (C.I. No. 61670) darstellt, mit einem Reinproduktgehalt von ca. 100 Gew.%; die Gehaltsbestimmung erfolgte durch Ausfärbung und photometrische Bestimmung der Farbstärke. Der Farbstoff entsprach in seinen coloristischen Eigenschaften dem nach den üblichen Methoden hergestellten Farbstoff C.I. Vat Red 21.

### Beispiel 7

In 250 kg Benzoesäuremethylester, entwässert wie in Beispiel 6, wurden bei Raumtemperatur 20 kg 1-Nitroanthrachinon-2-carbonsäure, 2,0 kg Tri-n-butylamin (entsprechend 0,16 mol pro Mol der Carbonsäure) und 12 kg Thionylchlorid eingebracht. Danach wurde innerhalb von einer Stunde auf 105°C erwärmt und eine Stunde bei dieser Temperatur gehalten. Nach Wiederabkühlung auf 80°C wurden 8,0 kg 1,4-Diaminoanthrachinon eingetragen. Anschließend wurde innerhalb von 3 Stunden auf 145°C erwärmt und 2 Stunden bei dieser Temperatur gehalten. Danach wurde die Temperatur auf 180°C erhöht und im Verlauf von 8 Stunden wurden 10 kg gasförmiges Ammoniak eingeleitet. Nach Abkühlung auf 80°C wurde das Produkt abfiltriert, mit Methanol und danach mit Wasser gewaschen und bei 120 °C im Vakuum getrocknet.

Man erhielt 26,5 kg des gleichen Farbstoffes wie in Beispiel 6 mit einem Reinproduktgehalt von ca. 100 Gew.%. Die Ausbeute betrug 96 %, die Raum-Zeit-Ausbeute lag bei 0,11 kg/l. Der Farbstoff entsprach in seinen coloristischen Eigenschaften dem nach den üblichen Methoden hergestellten Farbstoff C.I. Vat Red 21.

## Patentansprüche

1. Verfahren zur Herstellung von Anthrachinonderivaten der allgemeinen Formel I
(A¹-CO-NH-)ₙA² I
in der A¹ und A² Anthrachinon-Reste und/oder anthrachinoide Reste bedeuten und n für 1 oder 2 steht, durch Umsetzung der Anthrachinoncarbonsäuren oder ihrer Derivate der allgemeinen Formel II
A¹-COX II
in der X für eine Hydroxylgruppe oder ein Chlor- oder Bromatom steht, mit einem Aminoanthrachinon der allgemeinen Formel III
A²(NH₂)ₙ III
in Gegenwart einer tertiären Stickstoffbase, dadurch gekennzeichnet, daß man als tertiäre Stickstoffbase
(a) Trialkylamine mit drei gleichen oder verschiedenen nichtcyclischen Alkylresten mit der Ausnahme von Triethylamin im Falle der Umsetzung in Benzoesäuremethylester als Lösungsmittel,
(b) Trialkylamine mit 1, 2 oder 3 Cyclopentyl- und/oder Cyclohexylresten oder
(c) Aralkylamine mit einem Benzylrest
verwendet.

2. Verfahren zur Herstellung von Anthrachinonderivaten nach Anspruch 1, dadurch gekennzeichnet, daß man die tertiäre Stickstoffbase in einer Menge von 0,01 bis 1 mol pro Mol II einsetzt.

## Claims

1. A process for preparing anthraquinone derivatives of the general formula I
(A¹-CO-NH-)ₙA² I
where A¹ and A² are anthraquinone radicals and/or anthraquinonoid radicals and n is 1 or 2, by reacting the anthraquinonecarboxylic acids or anthraquinonecarboxylic acid derivatives of the general formula II
A¹-COX II
where X is a hydroxyl or a chlorine or bromine atom, with an aminoanthraquinone of the general formula III
A²(NH₂)ₙ III
in the presence of a tertiary nitrogen base, which comprises using as the tertiary nitrogen base
(a) a trialkylamine having three identical or different noncyclic alkyl radicals, provided that triethylamine shall be excluded if the reaction is carried out in methyl benzoate as solvent,
(b) a trialkylamine having 1, 2 or 3 cyclopentyl and/or cyclohexyl radicals, or
(c) an aralkylamine having a benzyl radical.

2. A process for preparing anthraquinone derivatives as claimed in claim 1, which comprises using the tertiary nitrogen base in an amount of from 0.01 to 1 mol per mole of II.

## Revendications

1. Procédé de préparation de dérivés de l'anthraquinone de la formule générale I
(A¹-CO-NH-)ₙA² I
dans laquelle A¹ et A² représentent des restes d'anthraquinone et/ou des restes anthraquinoïdiques et n est égal à 1 ou à 2, par la réaction d'acides anthraquinonecarboxyliques, ou de leurs dérivés, de la formule générale II
A¹-COX II
dans laquelle X représente un radical hydroxyle ou un atome de chlore ou de brome, avec une aminoanthraquinone de la formule générale III
A²(NH₂)ₙ III
en présence d'une base azotée tertiaire, caractérisé en ce qu'à titre de base azotée tertiaire, on utilise
(a) des trialkylamines qui comportent trois radicaux alkyle acycliques, identiques ou différents, à l'exclusion de la triéthylamine dans le cas de la réaction dans le benzoate de méthyle comme solvant,
(b) des trialkylamines qui comportent 1, 2 ou 3 radicaux cyclopentyle et/ou cyclohexyle, ou
(c) des aralkylamines qui comportent un radical benzyle.

2. Procédé de préparation de dérivés de l'anthraquinone selon la revendication 1, caractérisé en ce que l'on utilise la base azotée tertiaire en une proportion de 0,01 à 1 mole par mole du composé II.
